# FASCICULE DE BREVET EUROPEEN

(11) **EP 0 697 209 B1**
(45) Date de publication et mention de la délivrance du brevet: **09.10.1996**
(21) Numéro de dépôt: 95401499.9
(22) Date de dépôt: 23.06.1995
(51) Int. Cl.: A61K 7/13

(54) **Set, procédé, dispositif et composition de teinture des fibres kératiniques**
Set, Verfahren, Mittel und Zusammensetzung zur Färbung von Keratinfasern
Set, process, device and keratineous fibres dyeing composition

(30) Priorité: 22.07.1994 FR 9409118
(43) Date de publication de la demande: 21.02.1996
(73) Titulaire: L'OREAL, F-75008 Paris (FR)
(72) Inventeur: Samain, Henri, F-91570 Bievres (FR)
(74) Mandataire: Andral, Christophe André Louis

(56) Documents cités:
- EP-A- 0 446 131
- EP-A- 0 462 883
- WO-A-93/18738

## Description

L'invention est relative à un nouveau set de teinture des fibres kératiniques, et en particulier des fibres kératiniques humaines telles que les cheveux ainsi qu'au procédé de teinture des fibres kératiniques mettant en oeuvre ce set, et à un dispositif de teinture adéquat.

L'invention est également relative à une nouvelle composition pour la teinture.

Les colorants de la famille des indoles et des indolines sont bien connus pour leur utilisation en teinture des fibres kératiniques et notamment des cheveux humains.

Ces composés sont appliqués sur les cheveux selon un procédé mettant en oeuvre un agent révélateur dont le rôle est de produire la coloration des fibres par réaction avec le composé indolique et / ou indolinique.

Les brevets français FR-1 133 594 et FR-1 166 172 ainsi que la demande de brevet français FR-A-2 659 229 proposent des procédés de teinture à l'aide de 5,6-dihydroxyindole ou de composés indoliques, en mettant en oeuvre des cations métalliques jouant le rôle d'agent révélateur.

Parmi ces cations métalliques, sont cités les sels de manganèse. Ces derniers sont mis en oeuvre selon un procédé en deux étapes qui consiste dans un premier temps à appliquer le composé indolique sur les cheveux, à essorer puis, dans un deuxième temps, à appliquer une solution alcaline contenant un sel de manganèse. Selon ces documents, l'indole peut également être appliqué sur les cheveux en présence d'un agent alcalin, et après rinçage, on applique une solution contenant un sel de manganèse.

Ces procédés de teinture ne donnent pas entière satisfaction car les colorations obtenues avec les composés indoliques mis en oeuvre ne sont pas assez puissantes. De plus certains composés indoliques ne peuvent pas être révélés en utilisant ces procédés de teinture et par conséquent ils ne conduisent à aucune coloration des cheveux.

Afin de résoudre ces problèmes, la demanderesse a mis au point le set de teinture qui fait l'objet de l'invention.

L'invention a donc pour objet un set de teinture des fibres kératiniques et en particulier les fibres kératiniques humaines telles que les cheveux caractérisé par le fait qu'il est constitué de l'association d'une composition tinctoriale (A) contenant, dans un milieu approprié pour la teinture au moins un composé indolique et / ou indolinique et au moins un sel de manganèse, et d'une composition (B) contenant, dans un milieu approprié pour la teinture, au moins un agent alcalinisant et dans le lequel, lorsque que le pH de la composition tinctoriale (A) est inférieur à 7, le pH de la composition (B) est supérieur à 8,5 et lorsque le pH de la composition (A) est compris entre 7 et 9, le pH de la composition (B) est supérieur à 9,5.

Le pH de la composition tinctoriale (A) est de préférence inférieur à 7 et dans ce cas la composition tinctoriale (A) est nouvelle et constitue un autre objet de l'invention.

L'invention a également pour un procédé de teinture des fibres kératiniques et en particulier des fibres kératiniques humaines telles que les cheveux à l'aide du set de teinture tel que défini précédemment caractérisée par le fait que l'on applique dans un premier temps la composition tinctoriale (A) et dans un deuxième temps la composition (B) dans les conditions suivantes :
(i) : lorsque le pH de la composition tinctoriale (A) est inférieur à 7, le pH de la composition (B) est supérieur à 8,5 et
(ii) : lorsque le pH de la composition tinctoriale (A) est compris entre 7 et 9, le pH de la composition (B) est supérieure à 9,5.

La demanderesse a découvert que lorsque le composé indolique et / ou indolinique et le sel de manganèse sont appliqués simultanément sur les cheveux, et dans des conditions de pH telles que décrites précédemment, les colorations obtenues sont plus puissantes que celles de l'art antérieur et elles présentent de plus une excellente résistance aux divers traitements que peuvent subir les cheveux. De plus, le procédé de teinture de l'invention permet de révéler certains composés indoliques et en particulier certains monohydroxyindoles qui ne pouvaient être révélés de façon satisfaisante selon les procédés de l'état de la technique.

Les composés indoliques pouvant être utilisés dans la composition tinctoriale (A) définie ci-dessus peuvent répondre à la formule (I) suivante : 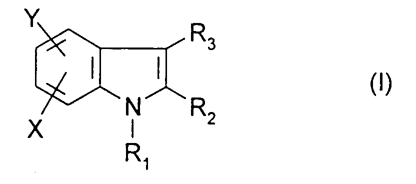
dans laquelle :
- R₁ et R₃ identiques ou différents représentent un atome d'hydrogène ou un radical alkyle en C₁-C₄ ;
- R₂ représente un atome d'hydrogène, un radical alkyle en C₁-C₄ ou -COOH ;
- X désigne un atome d'hydrogène, NH₂, OH, un radical alkyle en C₁-C₄ ou un radical alkoxy en C₁-C₄ ;
- Y désigne OH ou NH₂ ;
sous réserve que lorsque X désigne OH ou un radical alkyle, X occupe les positions 5, 6 ou 7 et est en position ortho par rapport à Y, ainsi que les sels de ces composés.

Parmi les composés indoliques de formule (I) préférentiels, on peut citer le 5,6-dihydroxyindole, le 2-méthyl 5,6-dihydroxyindole, le 3-méthyl 5,6-dihydroxyindole, le 4-hydroxyindole, le 2,3-diméthyl 5,6-dihydroxyindole, le 6-hydroxy 5-méthoxyindole, le 6-hydroxyindole, le 5-hydroxyindole, le 7-hydroxyindole, le 7-aminoindole, le 5-aminoindole, l'acide 5,6-dihydroxyindole 2-carboxylique, le 5-aminoindole, le 1-méthyl 5,6-dihydroxyindole et leurs sels.

Les composés indoliniques pouvant être utilisés dans la composition tinctoriale (A) définie ci-dessus peuvent répondre à la formule (II) suivante : 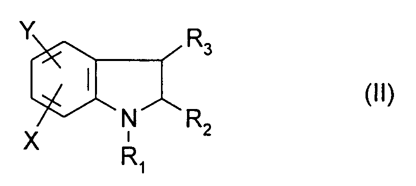
dans laquelle R₁, R₂, R₃, X et Y ont les mêmes significations que celles indiquées ci-dessus pour les composés de formule (I) .

Parmi les composés indoliniques de formule (II) préférentiels, on peut citer la 5,6-dihydroxyindoline, la N-méthyl 5,6-dihydroxyindoline, la N-éthyl 5,6-dihydroxyindoline, la N-butyl 5,6-dihydroxyindoline, la 4-hydroxy 5-méthoxyindoline, la 6-hydroxy 7-méthoxyindoline, la 6,7-dihydroxyindoline, la 4,5-dihydroxyindoline, la 5-méthoxy 6-hydroxyindoline et leurs sels.

Selon l'invention, les composés indoliques et / ou indoliniques sont de préférence présents à une concentration comprise entre 0,05 et 10 % en poids par rapport au poids total de la composition tinctoriale (A). Encore plus préférentiellement, cette concentration varie entre 0,2 et 5 % en poids par rapport au poids total de la composition tinctoriale (A).

Les sels de manganèse pouvant être utilisés dans la composition tinctoriale (A) présentent un degré d'oxydation égal à 2 ou à 3 et sont de préférence choisis parmi le diacétate de manganèse et ses hydrates tels que par exemple le diacétate de manganèse tétrahydrate, le dichlorure de manganèse et ses hydrates, les sulfates de manganèse, les carbonates de manganèse, les dihydrogénocarbonates de manganèse, l'acétylacétonate de manganèse, le triacétate de manganèse et ses hydrates, le trichlorure de manganèse.

Le diacétate de manganèse tétrahydrate est particulièrement préféré.

Selon l'invention, les sels de manganèse sont de préférence présents à une concentration comprise entre 0,002 et 5 % en poids d'équivalents métal par rapport au poids total de la composition tinctoriale (A). Encore plus préférentiellement, cette concentration est comprise entre 0,05 et 2 % en poids d'équivalents métal par rapport au poids total de la composition tinctoriale (A).

Selon une forme de réalisation préférée de l'invention, la composition tinctoriale (A) présente un pH compris entre 5 et 7.

Le pH de la composition tinctoriale (A) peut être ajusté à la valeur désirée à l'aide d'agents acidifiants classiques parmi lesquels on peut citer à titre d'exemple, l'acide orthophosphorique, l'acide lactique, l'acide acétique, l'acide tartrique, l'acide chlorhydrique et l'acide citrique.

Le milieu approprié pour la teinture est généralement un milieu aqueux constitué par de l'eau ou un mélange d'eau et d'un solvant organique utilisé pour solubiliser les composés qui ne seraient pas suffisamment solubles dans l'eau.

Parmi ces solvants, on peut citer à titre d'exemple, les alcanols inférieurs en C₁-C₄, tels que l'éthanol et l'isopropanol; le glycérol ; les glycols et éthers de glycols comme le 2-butoxyéthanol, le propylèneglycol, le monoéthyléther et le monométhyléther du diéthylèneglycol ; ainsi que les alcools aromatiques comme l'alcool benzilique ou le phénoxyéthanol ; les produits analogues et leurs mélanges.

Lorsqu'ils sont présents, les solvants représentent de préférence 1 à 40 % en poids du poids total de la composition tinctoriale (A) et encore plus préférentiellement de 5 à 30 % en poids.

La composition tinctoriale (A) peut également renfermer au moins un adjuvant choisi parmi les adjuvants utilisés classiquement dans les compositions pour la teinture des cheveux tels que les agents tensio-actifs anioniques, cationiques, nonioniques, amphotères, zwittérioniques ou leurs mélanges, les agents épaississants minéraux ou organiques, les agents antioxydants, les agents de pénétration, les agents séquestrants, les parfums, les tampons, les agents dispersants, les agents de conditionnement, les agents filmogènes, les agents conservateurs, les agents opacifiants, etc.

La composition tinctoriale (A) peut se présenter sous des formes diverses, telles que sous forme de liquide, de crème, de gel, de mousse ou sous toute autre forme appropriée pour réaliser une teinture des fibres kératiniques, et notamment des cheveux humains.

Les agents alcalinisants présents dans la composition (B) appliquée dans le deuxième temps sont de préférence choisis parmi les agents alcalinisants classiques tels que par exemple l'ammoniaque, les alcanolamines telles que par exemple les mono-, di- et triéthanolamines et leurs dérivés, les hydroxydes de sodium ou de potassium tels que par exemple la soude et la potasse, les alkylamines, les carbonates alcalins ou d'ammonium. La monoéthanolamine est particulièrement préférée.

La composition (B) contenant l'agent alcalinisant peut éventuellement renfermer un agent oxydant de façon à accélérer la révélation du composé indolique et / ou indolinique.

Cet agent oxydant peut être choisi parmi le peroxyde d'hydrogène, le peroxyde d'urée, les bromates de métaux alcalins, les persels tels que les perborates et persulfates. On utilise de préférence du peroxyde d'hydrogène dont le titre peut varier entre 1 et 40 volumes et de préférence entre 3 et 20 volumes.

Selon une forme de réalisation préférée de l'invention, la composition (B) contenant l'agent alcalinisant renferme un agent oxydant lorsque le composé indolique de formule (I) présent dans la composition tinctoriale (A) est un monohydroxyindole.

Selon le procédé de l'invention, on applique la composition tinctoriale (A) telle que définie précédemment sur les cheveux avec un temps de pause de préférence compris entre 1 et 30 minutes puis on applique la composition (B) contenant l'agent alcalinisant avec un temps de pause de préférence compris entre 1 et 30 minutes. Selon une forme de réalisation particulièrement préférée de l'invention, les temps de pause pour chaque composition sont compris entre 5 et 20 minutes.

Une étape d'essorage ou de rinçage peut, si on le souhaite, séparer l'application de la composition tinctoriale (A) de l'application de la composition (B) contenant l'agent alcalinisant.

La composition (B) contenant l'agent alcalinisant peut contenir les mêmes adjuvants que ceux définis pour la composition tinctoriale (A) et également se présenter sous des formes diverses telles que celles définies pour la composition tinctoriale (A).

L'invention a également pour objet un dispositif de teinture à plusieurs compartiments encore appelé "kit de teinture" ou "nécessaire de teinture" caractérisé par le fait qu'il comporte un premier compartiment renfermant la composition tinctoriale (A) telle que définie ci-dessus et un second compartiment renfermant la composition (B) contenant l'agent alcalinisant telle que définie ci-dessus. De tels dispositifs sont connus en eux-mêmes.

Les exemples qui suivent sont destinés à illustrer l'invention sans pour autant en limiter la portée.

### EXEMPLES DE TEINTURE

### EXEMPLES 1 à 4

On a préparé les compositions (A) et (B) suivantes :

### Composition (A) :

| | |
|---|---|
| - Ethanol | 10,0 g |
| - Gomme de guar hydroxypropylée vendue sous la dénomination commerciale JAGUAR HP 60 par la société MAYHALL | 0,8 g |
| - Akyl (C₈-C₁₀) polyglucoside en solution aqueuse à 60 % de matière active (MA) tamponné par du citrate d'ammonium (0,5 %) vendu sous la dénomination commerciale ORAMIX C6110 par la société SEPPIC | 8 g |
| **- Composé de formule (I) et / ou (II)** | **X g** |
| **- Diacétate de manganèse, tétrahydrate** | **1 g** |
| - Eau déminéralisée q.s.p. | 100 g |

### Composition (B) :

| | |
|---|---|
| - Diéthanolamide d'acide oléique | 0,8 g |
| - Glycérol | 0,4 g |
| - Mélange d'alcool cétylstéarylique et d'alcool cétylstéarylique polyoxyéthylèné à 33 moles d'oxyde d'éthylène (80 / 20) vendu sous la dénomination commerciale DEHSCONET 390 par la société TENSIA | 2 g |
| **- Monoéthanolamine** | **2 g** |
| **- Eau oxygénée** | **N volumes** |
| - Eau déminéralisée q.s.p. | 100 g |

La composition (A) a été appliquée pendant 15 minutes sur des cheveux gris naturels à 90 % de blancs. Après rinçage, on a appliqué la composition (B) pendant 10 minutes. Les cheveux ont ensuite été rincés et séchés.

Les résultats figurent dans le tableaux ci-après (teneurs en grammes) :

| **EXEMPLE** | **1** | **2** | **3** | **4** |
|---|---|---|---|---|
| **Composition (A)** | | | | |
| 5,6-dihydroxyindole | 1 | 1 | - | - |
| 4-hydroxyindole | - | - | 1 | - |
| 6-hydroxyindole | - | - | - | 1 |
| **pH (A)** | **6,5** | **6,5** | **6,5** | **6,5** |

| **Composition (B)** | | | | |
|---|---|---|---|---|
| Eau oxygénée (en volume) | - | 9 | 9 | 9 |
| **pH (B)** | **10,2** | **10,2** | **10,2** | **10,2** |
| **Nuance obtenue** | **gris très foncé** | **gris très foncé** | **cendré bleuté** | **doré mat** |

### EXEMPLE 5 à 10 COMPARATIFS

### EXEMPLE 5 ne faisant pas partie de l'invention

On a préparé la composition (A₅) suivante ne faisant pas partie de l'invention :

| | |
|---|---|
| - Ethanol | 10,0 g |
| - Gomme de guar hydroxypropylée vendue sous la dénomination commerciale JAGUAR HP 60 par la société MAYHALL | 0,8 g |
| - Akyl (C₈-C₁₀) polyglucoside en solution aqueuse à 60 % de matière active (MA) tamponné par du citrate d'ammonium (0,5 %) vendu sous la dénomination commerciale ORAMIX C6110 par la société SEPPIC | 8 g |
| **- 5,6-dihydroxyindole** | **1 g** |
| - Eau déminéralisée q.s.p | 100 g |

On a préparé la composition (B₅) suivante ne faisant pas partie de l'invention :

| | |
|---|---|
| - Diéthanolamide d'acide oléique | 0,8 g |
| - Glycérol | 0,4 g |
| - Mélange d'alcool cétylstéarylique et d'alcool cétylstéarylique polyoxyéthylèné à 33 moles d'oxyde d'éthylène (80 / 20) vendu sous la dénomination commerciale DEHSCONET 390 par la société TENSIA | 2 g |
| **- Monoéthanolamine** | **2 g** |
| **- Diacétate de manganèse, tétrahydrate** | **1 g** |
| - Eau déminéralisée q.s.p. | 100 g |

On a appliqué la composition (A₅) sur des cheveux gris naturels à 90 % de blancs pendant 15 minutes. Les cheveux ont ensuite été essorés puis on a appliqué la composition (B₅) pendant 10 minutes. Les cheveux ont ensuite été rincés et séchés.

Les résultats figurent dans le tableau récapitulatif situé après l'exemple 10.

### EXEMPLE 6 ne faisant pas partie de l'invention

On a préparé la composition (A₆) suivante ne faisant pas partie de l'invention :

| | |
|---|---|
| - Ethanol | 10,0 g |
| - Gomme de guar hydroxypropylée vendue sous la dénomination commerciale JAGUAR HP 60 par la société MAYHALL | 0,8 g |
| - Alkyl (C₈-C₁₀) polyglucoside en solution aqueuse à 60 % de matière active (MA) tamponné par du citrate d'ammonium (0,5 %) vendu sous la dénomination commerciale ORAMIX C6110 par la société SEPPIC | 8 g |
| **- 5,6-dihydroxyindole** | **1 g** |
| **- Monoéthanolamine** | **2 g** |
| - Eau déminéralisée q.s.p. | 100 g |

On a préparé la composition (B₆) suivante ne faisant pas partie de l'invention :

| | |
|---|---|
| - Diéthanolamide d'acide oléique | 0,8 g |
| - Glycérol | 0,4 g |
| - Mélange d'alcool cétylstéarylique et d'alcool cétylstéarylique polyoxyéthylèné à 33 moles d'oxyde d'éthylène (80 / 20) vendu sous la dénomination commerciale DEHSCONET 390 par la société TENSIA | 2 g |
| **- Diacétate de manganèse, tétrahydrate** | **1 g** |
| - Eau déminéralisée q.s.p. | 100 g |

On a appliqué la composition (A₆) sur des cheveux gris naturels à 90 % de blancs pendant 15 minutes. Les cheveux ont ensuite été rincés puis on a appliqué la composition (B₆) pendant 10 minutes. Les cheveux ont ensuite été rincés.

Les résultats figurent dans le tableau récapitulatif situé après l'exemple 10.

### EXEMPLE 7 ne faisant pas partie de l'invention

On a préparé la composition (A₇) suivante ne faisant pas partie de l'invention :

| | |
|---|---|
| - Ethanol | 10,0 g |
| - Gomme de guar hydroxypropylée vendue sous la dénomination commerciale JAGUAR HP 60 par la société MAYHALL | 0,8 g |
| - Alkyl (C₈-C₁₀) polyglucoside en solution aqueuse à 60 % de matière active (MA) tamponné par du citrate d'ammonium (0,5 %) vendu sous la dénomination commerciale ORAMIX C6110 par la société SEPPIC | 8 g |
| **- 5,6-dihydroxyindole** | **1 g** |
| **- Monoéthanolamine** | **2 g** |
| **- Diacétate de manganèse, tétrahydrate** | **1 g** |
| - Eau déminéralisée q.s.p | 100 g |

Il s'agit dans cet exemple d'un procédé en un seul temps ne faisant pas partie de l'invention.
On a appliqué la composition (A₇) sur des cheveux gris naturels à 90 % de blancs pendant 15 minutes puis on les a rincés et séchés.

Les résultats figurent dans le tableau récapitulatif situé après l'exemple 10.

### EXEMPLE 8 ne faisant pas partie de l'invention

On a préparé la composition (A₈) suivante ne faisant pas partie de l'invention :

| | |
|---|---|
| - Ethanol | 10,0 g |
| - Gomme de guar hydroxypropylée vendue sous la dénomination commerciale JAGUAR HP 60 par la société MAYHALL | 0,8 g |
| - Alkyl (C₈-C₁₀) polyglucoside en solution aqueuse à 60 % de matière active (MA) tamponné par du citrate d'ammonium (0,5 %) vendu sous la dénomination commerciale ORAMIX C6110 par la société SEPPIC | 8 g |
| **- 5,6-dihydroxyindole** | **1 g** |
| - Eau déminéralisée q.s.p. | 100 g |

On a préparé la composition (B₈) suivante faisant partie de l'invention :

| | |
|---|---|
| - Diéthanolamide d'acide oléique | 0,8 g |
| - Glycérol | 0,4 g |
| - Mélange d'alcool cétylstéarylique et d'alcool cétylstéarylique polyoxyéthylèné à 33 moles d'oxyde d'éthylène (80 / 20) vendu sous la dénomination commerciale DEHSCONET 390 par la société TENSIA | 2 g |
| **- Eau oxygénée** | **9 volumes** |
| **- Monoéthanolamine** | **2 g** |
| - Eau déminéralisée q.s.p. | 100 g |

On a appliqué la composition (A₈) sur des cheveux gris naturels à 90 % de blancs pendant 15 minutes. Les cheveux ont ensuite été essorés puis on a appliqué la composition (B₈) pendant 10 minutes. Les cheveux ont ensuite été rincés et séchés.

Les résultats figurent dans le tableau récapitulatif situé après l'exemple 10.

### EXEMPLE 9 faisant partie de l'invention

On a préparé la composition (A₉) suivante :

| | |
|---|---|
| - Ethanol | 10,0 g |
| - Gomme de guar hydroxypropylée vendue sous la dénomination commerciale JAGUAR HP 60 par la société MAYHALL | 0,8 g |
| - Alkyl (C₈-C₁₀) polyglucoside en solution aqueuse à 60 % de matière active (MA) tamponné par du citrate d'ammonium (0,5 %) vendu sous la dénomination commerciale ORAMIX C6110 par la société SEPPIC | 8 g |
| **- 5,6-dihydroxyindole** | **1 g** |
| **- Diacétate de manganèse, tétrahydrate** | **1 g** |
| - Eau déminéralisée q.s.p. | 100 g |

On a préparé la composition (B₉) suivante :

| | |
|---|---|
| - Diéthanolamide d'acide oléique | 0,8 g |
| - Glycérol | 0,4 g |
| - Mélange d'alcool cétylstéarylique et d'alcool cétylstéarylique polyoxyéthylèné à 33 moles d'oxyde d'éthylène (80 / 20) vendu sous la dénomination commerciale DEHSCONET 390 par la société TENSIA | 2 g |
| **Monoéthanolamine** | **2 g** |
| - Eau déminéralisée q.s.p. | 100 g |

La composition (A₉) a été appliquée pendant 15 minutes sur des cheveux gris naturels à 90 % de blancs. Après rinçage, on a appliqué la composition (B₉) pendant 10 minutes. Les cheveux ont ensuite été rincés et séchés.

Les résultats figurent dans le tableau récapitulatif situé après l'exemple 10.

### EXEMPLE 10 faisant partie de l'invention

On a préparé la composition (A₁₀) suivante :

Elle était identique à la composition (A₉)

On a préparé la composition (B₁₀) suivante :

Elle etait identique à la composition (B₉) sauf qu'elle contenait en plus 9 volumes d'eau oxygénée.

La composition (A₁₀) a été appliquée pendant 15 minutes sur des cheveux gris naturels à 90 % de blancs. Après rinçage, on a appliqué la composition (B₁₀) pendant 10 minutes. Les cheveux ont ensuite été rincés et séchés.

Les résultats figurent dans le tableau récapitulatif situé ci-après :

| **TABLEAU RECAPITULATIF DES EXEMPLES 5 à 10 :** | | | | | |
|---|---|---|---|---|---|
| **EXEMPLE** | **pH Composition (A)** | **pH Composition (B)** | **COLORATION OBTENUE** | | |
| | | | **L** | **a** | **b** |
| **5 (hors invention)** | 6,5 | 10,2 | **26,8** | 0,3 | -0,5 |
| **6 (hors invention)** | 6,5 | 10,2 | **27,9** | 0,3 | -0,6 |
| **7 (hors invention)** | 6,5 | 10,2 | **33,8** | -0,6 | -0,2 |
| **8 (hors invention)** | 6,5 | 10,2 | **24,0** | 0,8 | 1,0 |
| **9 (selon l'invention)** | 6,5 | 10,2 | **22,2** | 0,2 | 0,7 |
| **10 (selon l'invention)** | 6,5 | 10,2 | **22,0** | 0,2 | 0,5 |
| La couleur des mèches a été évaluée dans le système L. a. b.. | | | | | |

Selon ce système, L indique la clarté. Plus la valeur de L est élevée, plus la couleur est claire. Inversement, plus la valeur de L est faible, plus la couleur est foncée et donc puissante.

La teinte et la saturation sont exprimées par a et b.
a et b indiquent deux axes de couleur, a l'axe rouge vert et b l'axe jaune bleu.

Une valeur positive de a correspond à une teinte rouge d'autant plus saturée que la valeur absolue de a est élevée.

Une valeur négative de a correspond à une teinte verte d'autant plus saturée que la valeur absolue de a est élevée.

Une valeur positive de b correspond à une teinte jaune d'autant plus saturée que la valeur absolue de b est élevée.

Une valeur négative de b correspond à une teinte bleue d'autant plus saturée que la valeur absolue de b est élevée.

Des valeurs proches de zéro pour a ou b correspondent à des teintes grises.

Les résultats obtenus dans les exemples 5 à 10 montrent des valeurs de L plus faibles pour les teintures réalisées selon le procédé de l'invention (exemples 9 et 10). Les colorations obtenues aux exemples 9 et 10 sont donc plus puissantes que celles des exemples 5 à 8 selon l'art antérieur.

## Revendications

1. Set de teinture des fibres kératiniques et en particulier les fibres kératiniques humaines telles que les cheveux caractérisé par le fait qu'il est constitué de l'association d'une composition tinctoriale (A) contenant, dans un milieu approprié pour la teinture au moins un composé indolique et / ou indolinique et au moins un sel de manganèse, et d'une composition (B) contenant, dans un milieu approprié pour la teinture, au moins un agent alcalinisant et dans le lequel, lorsque que le pH de la composition tinctoriale (A) est inférieur à 7, le pH de la composition (B) est supérieur à 8,5 et lorsque le pH de la composition (A) est compris entre 7 et 9, le pH de la composition (B) est supérieur à 9,5.

2. Set de teinture selon la revendication 1, caractérisé par le fait que les composés indoliques présents dans la composition tinctoriale (A) sont choisis parmi les composés de formule (I) suivante : 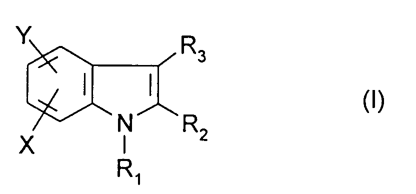 dans laquelle :
- R₁ et R₃ identiques ou différents représentent un atome d'hydrogène ou un radical alkyle en C₁-C₄ ;
- R₂ représente un atome d'hydrogène, un radical alkyle en C₁-C₄ ou -COOH ;
- X désigne un atome d'hydrogène, NH₂, OH, un radical alkyle en C₁-C₄ ou un radical alkoxy en C₁-C₄ ;
- Y désigne OH ou NH₂ ;
sous réserve que lorsque X désigne OH ou un radical alkyle, X occupe les positions 5, 6 ou 7 et est en position ortho par rapport à Y, ainsi que les sels de ces composés.

3. Set de teinture selon la revendication 2, caractérisé par le fait que les composés indoliques de formule (I) sont choisis parmi le 5,6-dihydroxyindole, le 2-méthyl 5,6-dihydroxyindole, le 3-méthyl 5,6-dihydroxyindole, le 4-hydroxyindole, le 2,3-diméthyl 5,6-dihydroxyindole, le 6-hydroxy 5-méthoxyindole, le 6-hydroxyindole, le 5-hydroxyindole, le 7-hydroxyindole, le 7-aminoindole, le 5-aminoindole, l'acide 5,6-dihydroxyindole 2-carboxylique, le 4-aminoindole, le 1-méthyl 5,6-dihydroxyindole et leurs sels.

4. Set de teinture selon l'une quelconque des revendications 1 à 3, caractérisé par le fait que les composés indoliniques présents dans la composition tinctoriale (A) sont choisis parmi les composés de formule (II) suivante : 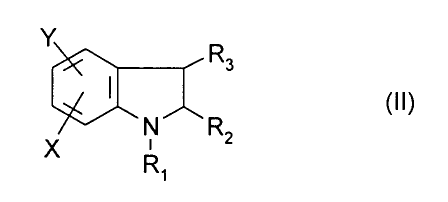 dans laquelle :
- R₁ et R₃ identiques ou différents représentent un atome d'hydrogène ou un radical alkyle en C₁-C₄ ;
- R₂ représente un atome d'hydrogène, un radical alkyle en C₁-C₄ ou -COOH ;
- X désigne un atome d'hydrogène, NH₂, OH, un radical alkyle en C₁-C₄ ou un radical alkoxy en C₁-C₄ ;
- Y désigne OH ou NH₂ ;
sous réserve que lorsque X désigne OH ou un radical alkyle, X occupe les positions 5, 6 ou 7 et est en position ortho par rapport à Y, ainsi que les sels de ces composés.

5. Set de teinture selon la revendication 4, caractérisé par le fait que les composés indoliniques de formule (II) sont choisis parmi la 5,6-dihydroxyindoline, la N-méthyl 5,6-dihydroxyindoline, la N-éthyl 5,6-dihydroxyindoline, la N-butyl 5,6-dihydroxyindoline, la 4-hydroxy 5-méthoxyindoline, la 6-hydroxy 7-méthoxyindoline, la 6,7-dihydroxyindoline, la 4,5-dihydroxyindoline, la 5-méthoxy 6-hydroxyindoline et leurs sels.

6. Set de teinture selon l'une quelconque des revendications 1 à 5, caractérisé par le fait que les composés indoliques et / ou indoliniques sont présents à une concentration comprise entre 0,05 et 10 % en poids par rapport au poids total de la composition tinctoriale (A).

7. Set de teinture selon la revendication 6, caractérisé par le fait que les composés indoliques et / ou indoliniques sont présents à une concentration comprise entre 0,2 et 5 % en poids par rapport au poids total de la composition tinctoriale (A).

8. Set de teinture selon l'une quelconque des revendications 1 à 7, caractérisé par le fait que les sels de manganèse, présents dans la composition tinctoriale (A), présentent un degré d'oxydation égal à 2 ou à 3.

9. Set de teinture selon l'une quelconque des revendications 1 à 8, caractérisé par le fait que les sels de manganèse sont choisis parmi le diacétate de manganèse et ses hydrates tels que par exemple le diacétate de manganèse tétrahydrate, le dichlorure de manganèse et ses hydrates, les sulfates de manganèse, les carbonates de manganèse, les dihydrogénocarbonates de manganèse, I'acétylacétonate de manganèse, le triacétate de manganèse et ses hydrates, le trichlorure de manganèse.

10. Set de teinture selon la revendication 9, caractérisé par le fait que le sel de manganèse est le diacétate de manganèse tétrahydrate.

11. Set de teinture selon l'une quelconque des revendications 1 à 10, caractérisé par le fait que les sels de manganèse sont présents à une concentration comprise entre 0,002 et 5 % en poids d'équivalents métal par rapport au poids total de la composition tinctoriale (A).

12. Set de teinture selon la revendication 11, caractérisé par le fait que les sels de manganèse sont présents à une concentration comprise entre 0,05 et 2 % en poids d'équivalents métal par rapport au poids total de la composition.

13. Set de teinture selon l'une quelconque des revendications 1 à 12, caractérisé par le fait que le milieu approprié pour la teinture est un milieu aqueux constitué par de l'eau ou un mélange d'eau et d'un solvant organique.

14. Set de teinture selon la revendication 13, caractérisé par le fait que les solvants utilisés sont choisis parmi les alcanols inférieurs en C₁-C₄, le glycérol; les glycols et éthers de glycols, les alcools aromatiques, les produits analogues et leurs mélanges.

15. Set de teinture selon l'une quelconque des revendications 1 à 14, caractérisé par le fait que l'agent alcalinisant est choisi l'ammoniaque, les alcanolamines, les hydroxydes de sodium ou de potassium, les alkylamines, les carbonates alcalins ou d'ammonium.

16. Set de teinture selon la revendication 15, caractérisé par le fait que l'agent alcalinisant est la monoéthanolamine.

17. Set de teinture selon l'une quelconque des revendications 1 à 16, caractérisé par le fait que la composition (B) renferme un agent oxydant de façon à accélérer la révélation du composé indolique et / ou indolinique.

18. Set de teinture selon la revendication 17, caractérisé par le fait que l'agent oxydant est choisi parmi le peroxyde d'hydrogène, le peroxyde d'urée, les bromates de métaux alcalins, les persels tels que les perborates et persulfates.

19. Set de teinture selon la revendication 18, caractérisé par le fait que l'agent oxydant est le peroxyde d'hydrogène.

20. Set de teinture selon l'une quelconque des revendications 1 à 19, caractérisé par le fait que la composition (B) renferme un agent oxydant lorsque le composé indolique de formule (I) présent dans la composition tinctoriale (A) est un monohydroxyindole.

21. Set de teinture selon l'une quelconque des revendications 1 à 20, caractérisé par le fait que la composition tinctoriale (A) et / ou la composition (B) se présentent sous forme de liquide, de crème, de gel, de mousse ou sous toute autre forme appropriée pour réaliser une teinture des fibres kératiniques, et notamment des cheveux humains.

22. Procédé de teinture des fibres kératiniques et particulier des fibres kératiniques humaines telles que les cheveux à l'aide du set de teinture tel que défini dans l'une quelconque des revendications 1 à 21, caractérisé par le fait qu'on applique sur ces fibres, dans un premier temps, la composition tinctoriale (A) et dans un deuxième temps, la composition (B) contenant l'agent alcalinisant puis on les rince.

23. Procédé selon la revendication 22, caractérisé par le fait qu'on applique la composition tinctoriale (A) sur les cheveux avec un temps de pause compris entre 1 et 30 minutes puis on applique la composition (B) contenant l'agent alcalinisant avec un temps de pause compris entre 1 et 30 minutes.

24. Procédé selon la revendication 23, caractérisé par le fait que les temps de pause pour chaque composition sont compris entre 5 et 20 minutes.

25. Procédé selon l'une quelconque des revendications 22 à 24, caractérisé par le fait qu'une étape d'essorage ou de rinçage sépare l'application de la composition tinctoriale (A) de l'application de la composition (B) contenant l'agent alcalinisant.

26. Dispositif de teinture à plusieurs compartiments caractérisé par le fait qu'il comporte un premier compartiment renfermant la composition tinctoriale (A) et un second compartiment renfermant la composition (B), lesdites compositions constituant le set tel que défini dans l'une quelconque des revendications 1 à 21.

27. Composition pour la teinture des fibres kératiniques et en particulier des fibres kératiniques humaines telles que les cheveux caractérisée par le fait qu'elle contient, dans un milieu approprié pour la teinture, au moins un composé indolique et / ou indolinique et au moins un sel de manganèse, le pH de cette composition étant inférieur à 7.

28. Composition selon la revendication 27, caractérisée par le fait que les composés indoliques répondent à la formule (I) suivante : 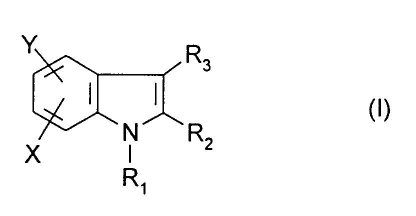 dans laquelle :
- R₁ et R₃ identiques ou différents représentent un atome d'hydrogène ou un radical alkyle en C₁-C₄ ;
- R₂ représente un atome d'hydrogène, un radical alkyle en C₁-C₄ ou -COOH ;
- X désigne un atome d'hydrogène, NH₂, OH, un radical alkyle en C₁-C₄ ou un radical alkoxy en C₁-C₄ ;
- Y désigne OH ou NH₂ ;
sous réserve que lorsque X désigne OH ou un radical alkyle, X occupe les positions 5, 6 ou 7 et est en position ortho par rapport à Y, ainsi que les sels de ces composés.

29. Composition selon la revendication 28, caractérisée par le fait que les composés indoliques de formule (I) sont choisis parmi le 5,6-dihydroxyindole, le 2-méthyl 5,6-dihydroxyindole, le 3-méthyl 5,6-dihydroxyindole, le 4-hydroxyindole, le 2,3-diméthyl 5,6-dihydroxyindole, le 6-hydroxy 5-méthoxyindole, le 6-hydroxyindole, le 5-hydroxyindole, le 7-hydroxyindole, le 7-aminoindole, le 5-aminoindole, l'acide 5,6-dihydroxyindole 2-carboxylique, le 4-aminoindole, le 1-méthyl 5,6-dihydroxyindole et leurs sels.

30. Composition selon l'une quelconque des revendications 27 à 29, caractérisée par le fait que les composés indoliniques répondent à la formule (II) suivante : 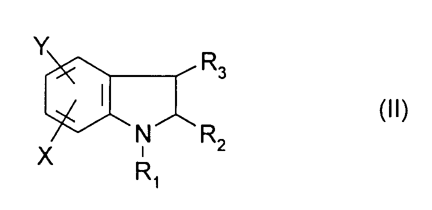 dans laquelle :
- R₁ et R₃ identiques ou différents représentent un atome d'hydrogène ou un radical alkyle en C₁-C₄ ;
- R₂ représente un atome d'hydrogène, un radical alkyle en C₁-C₄ ou -COOH ;
- X désigne un atome d'hydrogène, NH₂, OH, un radical alkyle en C₁-C₄ ou un radical alkoxy en C₁-C₄ ;
- Y désigne OH ou NH₂ ;
sous réserve que lorsque X désigne OH ou un radical alkyle, X occupe les positions 5, 6 ou 7 et est en position ortho par rapport à Y, ainsi que les sels de ces composés.

31. Composition selon la revendication 30, caractérisée par le fait que les composés indoliniques de formule (II) sont choisis parmi la 5,6-dihydroxyindoline, la N-méthyl 5,6-dihydroxyindoline, la N-éthyl 5,6-dihydroxyindoline, la N-butyl 5,6-dihydroxyindoline, la 4-hydroxy 5-méthoxyindoline, la 6-hydroxy 7-méthoxyindoline, la 6,7-dihydroxyindoline, la 4,5-dihydroxyindoline, la 5-méthoxy 6-hydroxyindoline et leurs sels.

32. Composition selon l'une quelconque des revendications 27 à 31, caractérisée par le fait que les composés indoliques et / ou indoliniques sontprésents à une concentration comprise entre 0,05 et 10 % en poids par rapport au poids total de la composition tinctoriale.

33. Composition selon la revendication 32, caractérisée par le fait que les composés indoliques et / ou indoliniques sont présents à une concentration comprise entre 0,2 et 5 % en poids par rapport au poids total de la composition tinctoriale.

34. Composition selon l'une quelconque des revendications 27 à 33, caractérisée par le fait que les sels de manganèse présentent un degré d'oxydation égal à 2 ou à 3.

35. Composition selon l'une quelconque des revendications 27 à 34, caractérisée par le fait que les sels de manganèse sont choisis parmi le diacétate de manganèse et ses hydrates tels que par exemple le diacétate de manganèse tétrahydrate, le dichlorure de manganèse et ses hydrates, les sulfates de manganèse, les carbonates de manganèse, les dihydrogénocarbonates de manganèse, I'acétylacétonate de manganèse, le triacétate de manganèse et ses hydrates, le trichlorure de manganèse.

36. Composition selon la revendication 35, caractérisée par le fait que le sel de manganèse est le diacétate de manganèse tétrahydrate.

37. Composition selon l'une quelconque des revendications 27 à 36, caractérisée par le fait que les sels de manganèse sont présents à une concentration comprise entre 0,002 et 5 % en poids d'équivalents métal par rapport au poids total de la composition tinctoriale.

38. Composition selon la revendication 37, caractérisée par le fait que les sels de manganèse sont présents à une concentration comprise entre 0,05 et 2 % en poids d'équivalents métal par rapport au poids total de la composition.

39. Composition selon l'une quelconque des revendications 27 à 38, caractérisée par le fait qu'elle présente un pH compris entre 5 et 7.

40. Composition selon l'une quelconque des revendications 27 à 39, caractérisée par le fait que le milieu approprié pour la teinture est un milieu aqueux constitué par de l'eau ou un mélange d'eau et d'un solvant organique.

41. Composition selon la revendication 40, caractérisée par le fait que les solvants utilisés sont choisis parmi les alcanols inférieurs en C₁-C₄, le glycérol; les glycols et éthers de glycols, les alcools aromatiques, les produits analogues et leurs mélanges.

42. Composition selon l'une quelconque des revendications 27 à 41, caractérisée par le fait qu'elle se présente sous forme de liquide, de crème, de gel, de mousse ou sous toute autre forme appropriée pour réaliser une teinture des fibres kératiniques, et notamment des cheveux humains.

## Claims

1. Set for dyeing keratinous fibres and in particular human keratinous fibres such as hair, characterized in that it consists of a combination of a dyeing composition (A) containing, in a medium appropriate for dyeing, at least one indole and/or indoline compound and at least one manganese salt and of a composition (B) containing, in a medium appropriate for dyeing, at least one basifying agent and in which, when the pH of the dyeing composition (A) is less than 7, the pH of the composition (B) is greater than 8.5 and, when the pH of the composition (A) is between 7 and 9, the pH of the composition (B) is greater than 9.5.

2. Dyeing set according to Claim 1, characterized in that the indole compounds present in the dyeing composition (A) are chosen from the compounds of following formula (I): 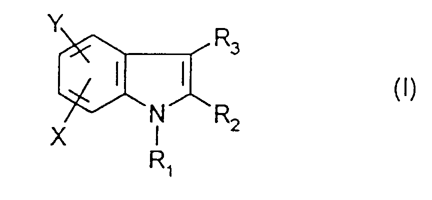 in which:
- R₁ and R₃, which are identical or different, represent a hydrogen atom or a C₁-C₄ alkyl radical;
- R₂ represents a hydrogen atom or a C₁-C₄ alkyl or -COOH radical;
- X denotes a hydrogen atom, NH₂, OH, a C₁-C₄ alkyl radical or a C₁-C₄ alkoxy radical;
- Y denotes OH or NH₂;
with the proviso that, when X denotes OH or an alkyl radical, X occupies the 5, 6 or 7 positions and is in the ortho position with respect to Y,
and the salts of these compounds.

3. Dyeing set according to Claim 2, characterized in that the indole compounds of formula (I) are chosen from 5,6-dihydroxyindole, 2-methyl-5,6-dihydroxyindole, 3-methyl-5,6-dihydroxyindole, 4-hydroxyindole, 2,3-dimethyl-5,6-dihydroxyindole, 6-hydroxy-5-methoxyindole, 6-hydroxyindole, 5-hydroxyindole, 7-hydroxyindole, 7-aminoindole, 5-aminoindole, 5,6-dihydroxyindole-2-carboxylic acid, 4-aminoindole and 1-methyl-5,6-dihydroxyindole, and their salts.

4. Dyeing set according to any one of Claims 1 to 3, characterized in that the indoline compounds present in the dyeing composition (A) are chosen from the compounds of following formula (II): 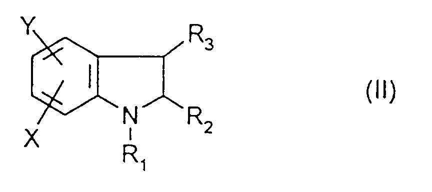 in which:
- R₁ and R₃, which are identical or different, represent a hydrogen atom or a C₁-C₄ alkyl radical;
- R₂ represents a hydrogen atom or a C₁-C₄ alkyl or -COOH radical;
- X denotes a hydrogen atom, NH₂, OH, a C₁-C₄ alkyl radical or a C₁-C₄ alkoxy radical;
- Y denotes OH or NH₂;
with the proviso that, when X denotes OH or an alkyl radical, X occupies the 5, 6 or 7 positions and is in the ortho position with respect to Y,
and the salts of these compounds.

5. Dyeing set according to Claim 4, characterized in that the indoline compounds of formula (II) are chosen from 5,6-dihydroxyindoline, N-methyl-5,6-dihydroxyindoline, N-ethyl-5,6-dihydroxyindoline, N-butyl-5,6-dihydroxyindoline, 4-hydroxy-5-methoxyindoline, 6-hydroxy-7-methoxyindoline, 6,7-dihydroxyindoline, 4,5-dihydroxyindoline and 5-methoxy-6-hydroxyindoline, and their salts.

6. Dyeing set according to any one of Claims 1 to 5, characterized in that the indole and/or indoline compounds are present at a concentration of between 0.05 and 10 % by weight with respect to the total weight of the dyeing composition (A).

7. Dyeing set according to Claim 6, characterized in that the indole and/or indoline compounds are present at a concentration of between 0.2 and 5 % by weight with respect to the total weight of the dyeing composition (A).

8. Dyeing set according to any one of Claims 1 to 7, characterized in that the manganese salts present in the dyeing composition (A) have an oxidation number equal to 2 or to 3.

9. Dyeing set according to any one of Claims 1 to 8, characterized in that the manganese salts are chosen from manganese diacetate and its hydrates, such as, for example, manganese diacetate tetrahydrate, manganese dichloride and its hydrates, manganese sulphates, manganese carbonates, manganese dihydrogencarbonates, manganese acetylacetonate, manganese triacetate and its hydrates and manganese trichloride.

10. Dyeing set according to Claim 9, characterized in that the manganese salt is manganese diacetate tetrahydrate.

11. Dyeing set according to any one of Claims 1 to 10, characterized in that the manganese salts are present at a concentration of between 0.002 and 5 % by weight of metal equivalents with respect to the total weight of the dyeing composition (A).

12. Dyeing set according to Claim 11, characterized in that the manganese salts are present at a concentration of between 0.05 and 2 % by weight of metal equivalents with respect to the total weight of the composition.

13. Dyeing set according to any one of Claims 1 to 12, characterized in that the medium appropriate for dyeing is an aqueous medium composed of water or a mixture of water and an organic solvent.

14. Dyeing set according to Claim 13, characterized in that the solvents used are chosen from lower C₁-C₄ alkanols, glycerol, glycols and glycol ethers, aromatic alcohols, analogous products and their mixtures.

15. Dyeing set according to any one of Claims 1 to 14, characterized in that the basifying agent is chosen from aqueous ammonia, alkanolamines, sodium hydroxide or potassium hydroxide, alkylamines or alkali metal or ammonium carbonates.

16. Dyeing set according to Claim 15, characterized in that the basifying agent is monoethanolamine.

17. Dyeing set according to any one of Claims 1 to 16, characterized in that the composition (B) contains an oxidizing agent, so as to accelerate the development of the indole and/or indoline compound.

18. Dyeing set according to Claim 17, characterized in that the oxidizing agent is chosen from hydrogen peroxide, urea hydrogen peroxide, alkali metal bromates and persalts such as perborates and persulphates.

19. Dyeing set according to Claim 18, characterized in that the oxidizing agent is hydrogen peroxide.

20. Dyeing set according to any one of Claims 1 to 19, characterized in that the composition (B) contains an oxidizing agent when the indole compound of formula (I) present in the dyeing composition (A) is a monohydroxyindole.

21. Dyeing set according to any one of Claims 1 to 20, characterized in that the dyeing composition (A) and/or the composition (B) are provided in the liquid, cream, gel or foam form or in any other form appropriate for carrying out dyeing of keratinous fibres and in particular of human hair.

22. Process for dyeing keratinous fibres, and in particular human keratinous fibres such as hair, using the dyeing set as defined in any one of Claims 1 to 21, characterized in that, in a first step, the dyeing composition (A) is applied to these fibres and, in a second step, the composition (B) containing the basifying agent is applied to these fibres and then they are rinsed.

23. Process according to Claim 22, characterized in that the dyeing composition (A) is applied to the hair with an exposure time of between 1 and 30 minutes and then the composition (B), containing the basifying agent, is applied with an exposure time of between 1 and 30 minutes.

24. Process according to Claim 23, characterized in that the exposure times for each composition are between 5 and 20 minutes.

25. Process according to any one of Claims 22 to 24, characterized in that a towel-drying or rinsing stage separates the application of the dyeing composition (A) from the application of the composition (B) containing the basifying agent.

26. Multi-compartment dyeing device, characterized in that it comprises a first compartment containing the dyeing composition (A) and a second compartment containing the composition (B), the said compositions constituting the set as defined in any one of Claims 1 to 21.

27. Composition for dyeing keratinous fibres and in particular human keratinous fibres such as hair, characterized in that it contains, in a medium appropriate for dyeing, at least one indole and/or indoline compound and at least one manganese salt, the pH of this composition being less than 7.

28. Composition according to Claim 27, characterized in that the indole compounds correspond to the following formula (I): 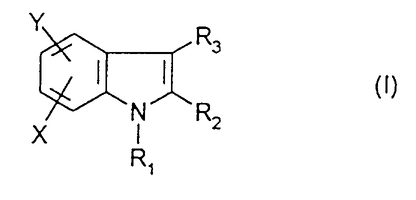 in which:
- R₁ and R₃, which are identical or different, represent a hydrogen atom or a C₁-C₄ alkyl radical;
- R₂ represents a hydrogen atom or a C₁-C₄ alkyl or -COOH radical;
- X denotes a hydrogen atom, NH₂, OH, a C₁-C₄ alkyl radical or a C₁-C₄ alkoxy radical;
- Y denotes OH or NH₂;
with the proviso that, when X denotes OH or an alkyl radical, X occupies the 5, 6 or 7 positions and is in the ortho position with respect to Y,
and the salts of these compounds.

29. Composition according to Claim 28, characterized in that the indole compounds of formula (I) are chosen from 5,6-dihydroxyindole, 2-methyl-5,6-dihydroxyindole, 3-methyl-5,6-dihydroxyindole, 4-hydroxyindole, 2,3-dimethyl-5,6-dihydroxyindole, 6-hydroxy-5-methoxyindole, 6-hydroxyindole, 5-hydroxyindole, 7-hydroxyindole, 7-aminoindole, 5-aminoindole, 5,6-dihydroxyindole-2-carboxylic acid, 4-aminoindole and 1-methyl-5,6-dihydroxyindole, and their salts.

30. Composition according to any one of Claims 27 to 29, characterized in that the indoline compounds correspond to the following formula (II): 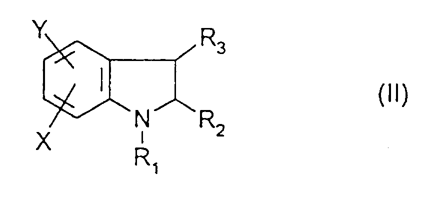 in which:
- R₁ and R₃, which are identical or different, represent a hydrogen atom or a C₁-C₄ alkyl radical;
- R₂ represents a hydrogen atom or a C₁-C₄ alkyl or -COOH radical;
- X denotes a hydrogen atom, NH₂, OH, a C₁-C₄ alkyl radical or a C₁-C₄ alkoxy radical;
- Y denotes OH or NH₂;
with the proviso that, when X denotes OH or an alkyl radical, X occupies the 5, 6 or 7 positions and is in the ortho position with respect to Y,
and the salts of these compounds.

31. Composition according to Claim 30, characterized in that the indoline compounds of formula (II) are chosen from 5,6-dihydroxyindoline, N-methyl-5,6-dihydroxyindoline, N-ethyl-5,6-dihydroxyindoline, N-butyl-5,6-dihydroxyindoline, 4-hydroxy-5-methoxyindoline, 6-hydroxy-7-methoxyindoline, 6,7-dihydroxyindoline, 4,5-dihydroxyindoline and 5-methoxy-6-hydroxyindoline, and their salts.

32. Composition according to any one of Claims 27 to 31, characterized in that the indole and/or indoline compounds are present at a concentration of between 0.05 and 10 % by weight with respect to the total weight of the dyeing composition.

33. Composition according to Claim 32, characterized in that the indole and/or indoline compounds are present at a concentration of between 0.2 and 5 % by weight with respect to the total weight of the dyeing composition.

34. Composition according to any one of Claims 27 to 33, characterized in that the manganese salts have an oxidation number equal to 2 or to 3.

35. Composition according to any one of Claims 27 to 34, characterized in that the manganese salts are chosen from manganese diacetate and its hydrates, such as, for example, manganese diacetate tetrahydrate, manganese dichloride and its hydrates, manganese sulphates, manganese carbonates, manganese dihydrogencarbonates, manganese acetylacetonate, manganese triacetate and its hydrates and manganese trichloride.

36. Composition according to Claim 35, characterized in that the manganese salt is manganese diacetate tetrahydrate.

37. Composition according to any one of Claims 27 to 36, characterized in that the manganese salts are present at a concentration of between 0.002 and 5 % by weight of metal equivalents with respect to the total weight of the dyeing composition.

38. Composition according to Claim 37, characterized in that the manganese salts are present at a concentration of between 0.05 and 2 % by weight of metal equivalents with respect to the total weight of the composition.

39. Composition according to any one of Claims 27 to 38, characterized in that it has a pH of between 5 and 7.

40. Composition according to any one of Claims 27 to 39, characterized in that the medium appropriate for dyeing is an aqueous medium composed of water or a mixture of water and an organic solvent.

41. Composition according to Claim 40, characterized in that the solvents used are chosen from lower C₁-C₄ alkanols, glycerol, glycols and glycol ethers, aromatic alcohols, analogous products and their mixtures.

42. Composition according to any one of Claims 27 to 41, characterized in that it is provided in the liquid, cream, gel or foam form or in any other form appropriate for carrying out dyeing of keratinous fibres and in particular of human hair.

## Patentansprüche

1. Set zum Färben von Keratinfasern und insbesondere von menschlichen Keratinfasern, wie dem Haar, dadurch gekennzeichnet, daß es aus einer Kombination einer Färbemittelzusammensetzung (A), die in einem zum Färben geeigneten Medium mindestens eine Indolverbindung und/oder Indolinverbindung und mindestens ein Mangansalz enthält, und einer Zusammensetzung (B) besteht, die in einem zum Färben geeigneten Medium mindestens ein Mittel zum Alkalischmachen enthält, wobei der pH-Wert der Zusammensetzung (B) einen Wert über 8,5 aufweist, wenn der pH-Wert der Färbemittelzusammensetzung (A) unter 7 liegt, und der pH-Wert der Zusammensetzung (B) einen Wert über 9,5 aufweist, wenn die Zusammensetzung (A) einen pH-Wert im Bereich von 7 bis 9 aufweist.

2. Set zum Färben nach Anspruch 1, dadurch gekennzeichnet, daß die in der färbemittelzusammensetzung (A) vorliegenden Indolverbindungen unter Verbindungen der folgenden Formel I ausgewählt sind: 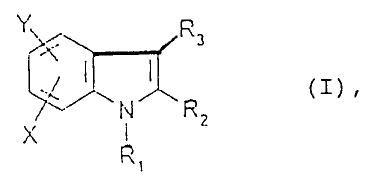 worin bedeuten:
R₁ und R₃, die gleich oder voneinander verschieden sind, Wasserstoff oder C₁₋₄-Alkyl,
R₂ Wasserstoff, C₁₋₄-Alkyl oder -COOH,
X Wasserstoff, NH₂, OH, C₁₋₄-Alkyl oder C₁₋₄-Alkoxy,
Y OH oder NH₂,
mit der Maßgabe, daß X in 5-, 6- oder 7-Position und in Bezug auf Y in o-Position substituiert ist, wenn X OH oder Alkyl bedeutet
sowie die Salze dieser Verbindungen.

3. Set zum Färben nach Anspruch 2, dadurch gekennzeichnet, daß die Indolverbindungen der Formel I unter 5,6-Dihydroxyindol, 2-Methyl-5,6-dihydroxyindol, 3-Methyl-5,6-dihydroxyindol, 4-Hydroxyindol, 2,3-Dimethyl-5,6-dihydroxyindol, 6-Hydroxy-5-methoxyindol, 6-Hydroxyindol, 5-Hydroxyindol, 7-Hydroxyindol, 7-Aminoindol, 5-Aminoindol, 2-Carboxy-5,6-dihydroxyindol, 4-Aminoindol, 1-Methyl-5,6-dihydroxyindol und ihren Salzen ausgewählt sind.

4. Set zum Färben nach einem der Ansprüche 1 bis 3, dadurch gekennzeichnet, daß die in der Färbemittelzusammensetzung (A) vorliegenden Indolinverbindungen unter den Verbindungen der folgenden Formel II ausgewählt sind: 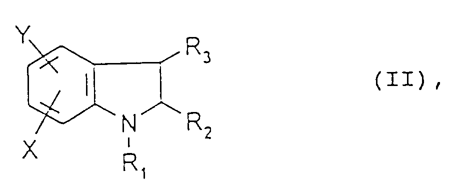 worin bedeuten:
R₁ und R₃, die gleich oder voneinander verschieden sind, Wasserstoff oder C₁₋₄-Alkyl,
R₂ Wasserstoff, C₁₋₄-Alkyl oder -COOH,
X Wasserstoff, NH₂, OH, C₁₋₄-Alkyl oder C₁₋₄-Alkoxy,
Y OH oder NH₂,
mit der Maßgabe, daß X in 5-, 6- oder 7-Position und in Bezug auf Y in o-Position substituiert ist, wenn X OH oder Alkyl bedeutet,
sowie die Salze dieser Verbindungen.

5. Set zum Färben nach Anspruch 4, dadurch gekennzeichnet, daß die Indolinverbindungen der Formel II unter 5,6-Dihydroxyindolin, N-Methyl-5,6-dihydroxyindolin, N-Ethyl-5,6-dihydroxyindolin, N-Butyl-5,6-dihydroxyindolin, 4-Hydroxy-5-methoxyindolin, 6-Hydroxy-7-methoxyindolin, 6,7-Dihydroxyindolin, 4,5-Dihydroxyindolin, 5-Methoxy-6-hydroxyindolin und ihren Salzen ausgewählt sind.

6. Set zum Färben nach einem der Ansprüche 1 bis 5, dadurch gekennzeichnet, daß die Indolverbindungen und/oder Indolinverbindungen in einer Konzentration von 0,05 bis 10 Gew.-%, bezogen auf das Gesamtgewicht der Färbemittelzusammensetzung (A), vorliegen.

7. Set zum Färben nach Anspruch 6, dadurch gekennzeichnet, daß die Indolverbindungen und/oder Indolinverbindungen in einer Konzentration von 0,2 bis 5 Gew.-%, bezogen auf das Gesamtgewicht der Färbemittelzusammensetzung (A), vorliegen.

8. Set zum Färben nach einem der Ansprüche 1 bis 7, dadurch gekennzeichnet, daß die in der Färbemittelzusammensetzung (A) vorliegenden Mangansalze eine Oxidationsstufe von 2 oder 3 aufweisen.

9. Set zum Färben nach einem der Ansprüche 1 bis 8, dadurch gekennzeichnet, daß die Mangansalze unter Mangandiacetat und seinen Hydraten, wie beispielsweise Mangandiacetattetrahydrat, Mangandichlorid und seinen Hydraten, Mangansulfaten, Mangancarbonaten, Mangandihydrogencarbonaten, Manganacetylacetonat, Mangantriacetat und seinen Hydraten und Mangantrichlorid ausgewählt sind.

10. Set zum Färben nach Anspruch 9, dadurch gekennzeichnet, daß das Mangansalz das Mangandiacetattetrahydrat ist.

11. Set zum Färben nach einem der Ansprüche 1 bis 10, dadurch gekennzeichnet, daß die Mangansalze in einer Konzentration von 0,002 bis 5 Gew.-%, ausgedrückt als Metall, bezogen auf das Gesamtgewicht der Färbemittelzusammensetzung (A), vorliegen.

12. Set zum Färben nach Anspruch 11, dadurch gekennzeichnet, daß die Mangansalze in einer Konzentration von 0,05 bis 2 Gew.-%, ausgedrückt als Metall, bezogen auf das Gesamtgewicht der Zusammensetzung, vorliegen.

13. Set zum Färben nach einem der Ansprüche 1 bis 12, dadurch gekennzeichnet, daß das zum Färben geeignete Medium ein wäßriges Medium ist, das aus Wasser oder einem Gemisch aus Wasser und einem organischen Lösungsmittel besteht.

14. Set zum Färben nach Anspruch 13, dadurch gekennzeichnet, daß die verwendeten Lösungsmittel unter niederen C₁₋₄-Alkanolen, Glycerin, Glykolen und Glykolethern, aromatischen Alkoholen, analogen Produkten und ihren Gemischen ausgewählt sind.

15. Set zum Färben nach einem der Ansprüche 1 bis 14, dadurch gekennzeichnet, daß das Mittel zum Alkalischmachen unter Ammoniak, Alkanolaminen, Natriumhydroxid, Kaliumhydroxid, Alkylaminen, Alkalicarbonaten oder Ammoniumcarbonaten ausgewählt ist.

16. Set zum Färben nach Anspruch 15, dadurch gekennzeichnet, daß das Mittel zum Alkalischmachen Monoethanolamin ist.

17. Set zum Färben nach einem der Ansprüche 1 bis 16, dadurch gekennzeichnet, daß die Zusammensetzung (B) ein Oxidationsmittel enthält, um das Entwickeln der Indolverbindung und/oder Indolinverbindung zu beschleunigen.

18. Set zum Färben nach Anspruch 17, dadurch gekennzeichnet, daß das Oxidationsmittel unter Wasserstoffperoxid, Harnstoffperoxid, Alkalibromaten und Salzen von Persäuren, wie Perboraten und Persulfaten, ausgewählt ist.

19. Set zum Färben nach Anspruch 18, dadurch gekennzeichnet, daß das Oxidationsmittel Wasserstoffperoxid ist.

20. Set zum Färben nach einem der Ansprüche 1 bis 19, dadurch gekennzeichnet, daß die Zusammensetzung (B) ein Oxidationsmittel enthält, wenn die in der Färbemittelzusammensetzung (A) vorliegende Indolverbindung der Formel (I) ein Monohydroxyindol ist.

21. Set zum Färben nach einem der Ansprüche 1 bis 20, dadurch gekennzeichnet, daß die Färbemittelzusammensetzung (A) und/oder die Zusammensetzung (B) in flüssiger Form, als Creme, Gel oder Schaum oder in beliebigen weiteren Formen vorliegen, die zum Färben von Keratinfasern, und insbesondere von menschlichem Haar, geeignet sind.

22. Verfahren zum Färben von Keratinfasern und insbesondere menschlichen Keratinfasern, wie dem Haar, mit dem in einem der Ansprüche 1 bis 21 definierten Set zum Färben, dadurch gekennzeichnet, daß auf die Fasern zu einem ersten Zeitpunkt die Färbemittelzusammensetzung (A) und zu einem zweiten Zeitpunkt die Zusammensetzung (B), die das Mittel zum Alkalischmachen enthält, aufgetragen wird, und anschließend die Haare gespült werden.

23. Verfahren nach Anspruch 22, dadurch gekennzeichnet, daß die Färbemittelzusammensetzung (A) auf die Haare mit einer Einwirkdauer von 1 bis 30 min und anschließend die Zusammensetzung (B), die das Mittel zum Alkalischmachen enthält, mit einer Einwirkdauer von 1 bis 30 min aufgetragen wird.

24. Verfahren nach Anspruch 23, dadurch gekennzeichnet, daß die Einwirkdauer für jede Zusammensetzung im Bereich von 5 bis 20 min liegt.

25. Verfahren nach einem der Ansprüche 22 bis 24, dadurch gekennzeichnet, daß zwischen der Anwendung der Färbemittelzusammensetzung (A) und der Anwendung der Zusammensetzung (B), die das Mittel zum Alkalischmachen enthält, ein Schritt des Auswringens oder Spülens liegt.

26. Vorrichtung zum Färben mit mehreren Abteilungen, dadurch gekennzeichnet, daß sie in einer ersten Abteilung die Färbemittelzusammensetzung (A) und in einer zweiten Abteilung die Zusammensetzung (B) enthält, wobei diese Zusammensetzungen das nach einem der Ansprüche 1 bis 21 definierte Set bilden.

27. Zusammensetzung zum Färben von Keratinfasern und insbesondere menschlichen Keratinfasern, wie dem Haar, dadurch gekennzeichnet, daß sie in einem zum Färben geeigneten Medium mindestens eine Indolverbindung und/oder Indolinverbindung und mindestens ein Mangansalz enthält, wobei der pH-Wert dieser Zusammensetzung unter 7 liegt.

28. Zusammensetzung nach Anspruch 27, dadurch gekennzeichnet, daß die Indolverbindung der folgenden Formel I entsprechen: 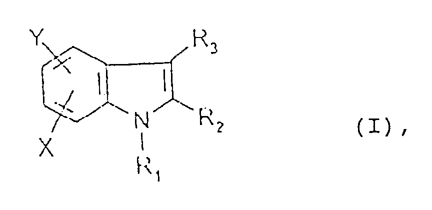 worin bedeuten:
R₁ und R₃, die gleich oder voneinander verschieden sind, Wasserstoff oder C₁₋₄-Alkyl,
R₂ Wasserstoff, C₁₋₄-Alkyl oder -COOH,
X Wasserstoff, NH₂, OH, C₁₋₄-Alkyl oder C₁₋₄-Alkoxy,
Y OH oder NH₂,
mit der Maßgabe, daß X in 5-, 6- oder 7-Position und in Bezug auf Y in o-Position substituiert ist, wenn X OH oder Alkyl bedeutet
sowie die Salze dieser Verbindungen.

29. Zusammensetzung nach Anspruch 28, dadurch gekennzeichnet, daß die Indolverbindungen der Formel I unter 5,6-Dihydroxyindol, 2-Methyl-5,6-dihydroxyindol, 3-Methyl-5,6-dihydroxyindol, 4-Hydroxyindol, 2,3-Dimethyl-5,6-dihydroxyindol, 6-Hydroxy-5-methoxyindol, 6-Hydroxyindol, 5-Hydroxyindol, 7-Hydroxyindol, 7-Aminoindol, 5-Aminoindol, 2-Carboxy-5,6-dihydroxyindol, 4-Aminoindol, 1-Methyl-5,6-dihydroxyindol und ihren Salzen ausgewählt sind.

30. Zusammensetzung nach einem der Ansprüche 27 bis 29, dadurch gekennzeichnet, daß die Indolinverbindungen der folgenden Formel II entsprechen: 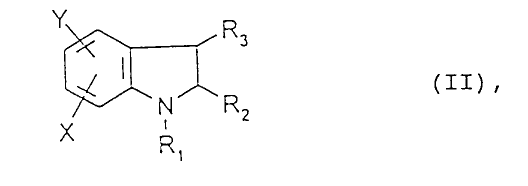 worin bedeuten:
R₁ und R₃, die gleich oder voneinander verschieden sind, Wasserstoff oder C₁₋₄-Alkyl,
R₂ Wasserstoff, C₁₋₄-Alkyl oder -COOH,
X Wasserstoff, NH₂, OH, C₁₋₄-Alkyl oder C₁₋₄-Alkoxy,
Y OH oder NH₂,
mit der Maßgabe, daß X in 5-, 6- oder 7-Position und in Bezug auf Y in o-Position substituiert ist, wenn X OH oder Alkyl bedeutet,
sowie die Salze dieser Verbindungen.

31. Zusammensetzung nach Anspruch 30, dadurch gekennzeichnet, daß die Indolinverbindungen der Formel II unter 5,6-Dihydroxyindolin, N-Methyl-5,6-dihydroxyindolin, N-Ethyl-5,6-dihydroxyindolin, N-Butyl-5,6-dihydroxyindolin, 4-Hydroxy-5-methoxyindolin, 6-Hydroxy-7-methoxyindolin, 6,7-Dihydroxyindolin, 4,5-Dihydroxyindolin, 5-Methoxy-6-hydroxyindolin und ihren Salzen ausgewählt sind.

32. Zusammensetzung nach einem der Ansprüche 27 bis 31, dadurch gekennzeichnet, daß die Indolverbindungen und/oder Indolinverbindungen in einer Konzentration von 0,05 bis 10 Gew.-%, bezogen auf das Gesamtgewicht der Färbemittelzusammensetzung, vorliegen.

33. Zusammensetzung nach Anspruch 32, dadurch gekennzeichnet, daß die Indolverbindungen und/oder Indolinverbindungen in einer Konzentration von 0,2 bis 5 Gew.-%, bezogen auf das Gesamtgewicht der Färbemittelzusammensetzung, vorliegen.

34. Zusammensetzung nach einem der Ansprüche 27 bis 33, dadurch gekennzeichnet, daß die Mangansalze eine Oxidationsstufe von 2 oder 3 aufweisen.

35. Zusammensetzung nach einem der Ansprüche 27 bis 34, dadurch gekennzeichnet, daß die Mangansalze unter Mangandiacetat und seinen Hydraten, wie beispielsweise Mangandiacetattetrahydrat, Mangandichlorid und seinen Hydraten, Mangansulfaten, Mangancarbonaten, Mangandihydrogencarbonaten, Manganacetylacetonat, Mangantriacetat und seinen Hydraten und Mangantrichlorid ausgewählt sind.

36. Zusammensetzung nach Anspruch 35, dadurch gekennzeichnet, daß das Mangansalz das Mangandiacetattetrahydrat ist.

37. Zusammensetzung nach einem der Ansprüche 27 bis 36, dadurch gekennzeichnet, daß die Mangansalze in einer Konzentration von 0,002 bis 5 Gew.-% , ausgedrückt als Metall, bezogen auf das Gesamtgewicht der Färbemittelzusammensetzung, vorliegen.

38. Zusammensetzung nach Anspruch 37, dadurch gekennnzeichnet, daß die Mangansalze in einer Konzentration von 0,05 bis 2 Gew.-%, ausgedrückt als Metall, bezogen auf das Gesamtgewicht der Zusammensetzung, vorliegen.

39. Zusammensetzung nach einem der Ansprüche 27 bis 38, dadurch gekennzeichnet, daß sie einen pH-Wert im Bereich von 5 bis 7 aufweist.

40. Zusammensetzung nach einem der Ansprüche 27 bis 39, dadurch gekennzeichnet, daß das zum Färben geeignete Medium ein wäßriges Medium ist, das aus Wasser oder einem Gemisch aus Wasser und einem organischen Lösungsmittel besteht.

41. Zusammensetzung nach Anspruch 40, dadurch gekennzeichnet, daß die verwendeten Lösungsmittel unter niederen C₁₋₄-Alkanolen, Glycerin, Glykolen und Glykolethern, aromatischen Alkoholen, analogen Produkten und ihren Gemischen ausgewählt sind.

42. Zusammensetzung nach einem der Ansprüche 27 bis 41, dadurch gekennzeichnet, daß sie in flüssiger Form, als Creme, Gel oder Schaum oder allen beliebigen weiteren Formen vorliegt, die zum Färben von Keratinfasern und insbesondere menschlichem Haar, geeignet sind.
